# EUROPÄISCHE PATENTSCHRIFT

(11) **EP 1 484 038 B1**
(45) Veröffentlichungstag und Bekanntmachung des Hinweises auf die Patenterteilung: **01.08.2007**
(21) Anmeldenummer: 04012159.2
(22) Anmeldetag: 22.05.2004
(51) Int. Cl.: A61F 5/00

(54) **Einrichtung zur Erzeugung einer künstlichen Verengung im Gastro-Intestinal-Trakt**
Band to produce an artificial reduction in the gastrointestinal tract
Anneau pour produire une constriction artificielle du tract gastro-intestinal

(30) Priorität: 04.06.2003 AT 8612003
(43) Veröffentlichungstag der Anmeldung: 08.12.2004
(73) Patentinhaber: AMI (Agency for Medical Innovations GmbH), 6840 Götzis (AT)
(72) Erfinder: Egle, Walter, 6842 Koblach (AT)
(74) Vertreter: Hofmann, Ralf U.

(56) Entgegenhaltungen:
- EP-A- 0 702 529
- WO-A-03/020183
- FR-A- 2 834 202

## Beschreibung

Die Erfindung betrifft eine Einrichtung zur Erzeugung einer künstlichen Verengung im Gastro-Intestinal-Trakt mit einem ringförmig um den jeweiligen Teil des Gastro-Intestinal-Traktes legbaren Band und einer Verschlusseinrichtung zum Verbinden der Endbereiche des ringförmig um den Teil des Gastro-Intestinal-Traktes gelegten Bandes, welches im geschlossenen Zustand der Verschlusseinrichtung eine Durchtrittsöffnung umschließt, wobei das Band eine mit einem Füllmedium befüllbare Hohlkammer aufweist, die sich über zumindest einen Großteil der Länge des Bandes erstreckt und auf ihrer der Durchtrittsöffnung zugewandten Seite von einer Membran begrenzt ist, mittels der durch Befüllung der Hohlkammer die Durchtrittsöffnung verkleinerbar ist.

Eine solche Einrichtung ist beispielsweise in Form eines Magenbandes aus der EP 0 702 529 B1 bekannt. Die Einrichtung weist ein um den Mageneingang legbares Band auf, welches mit einer längs verlaufenden inneren Hohlkammer ausgebildet ist. Zum Verschließen des um den Mageneingang ringförmig gelegten Bandes weist dieses eine Verschlusseinrichtung mit einem am einen Ende des Bandes angeordneten und eine Einstecköffnung aufweisenden ersten Verschlussteil und einem am anderen Ende des Bandes angeordneten, durch die Einstecköffnung einführbaren und gegenüber dieser verrastbaren zweiten Verschlussteil. Zur Verengung des Durchlassquerschnittes der Durchtrittsöffnung des Bandes und somit des Mageneinganges wird die Hohlkammer des Bandes mit einem Füllmedium befüllt, wobei die Menge des Füllmediums vom gewünschten Durchlassquerschnitt abhängt. Zur Befüllung des Bandes mit dem Füllmedium ist ein mit dem Band verbundener Injektionsport vorgesehen, der unter die Haut des Patienten implantiert wird.

Neben einer Ausbildung als Magenband kann eine Einrichtung dieser Art insbesondere auch als Analband zum Verschluss eines, gegebenenfalls künstlichen, Anus ausgebildet sein.

Ein Problem bei solchen Einrichtungen besteht darin, dass diese früher oder später im Laufe ihres Gebrauchs leck werden können, so dass ihre Funktion nicht mehr gegeben ist. Es ist in der Folge eine operative Entfernung und Ersetzung des Bandes erforderlich, was mit einer entsprechenden Belastung des Patienten verbunden ist. Solche Lecks treten in der Praxis insbesondere in der das Band zur Durchtrittsöffnung hin begrenzenden Membran auf. Solche Lecks können beispielsweise durch Materialermüdung im Laufe des Gebrauchs oder durch eine Überfüllung des Bandes auftreten. Für sogenannte "frühe" Lecks, die bis etwa ein Jahr nach dem Einsetzen des Bandes auftreten, sind meist Verletzungen verantwortlich, die bei der operativen, insbesondere laparoskopischen, Platzierung des Bandes durch ein chirurgisches Instrument erfolgt sind.

Aus der WO 03/020183 A1 ist weiters ein Magenband bekannt, welches einen Bandabschnitt aufweist, mit dem die Magenöffnung einstellbar ist, insbesondere mittels eines den Bandabschnitt zusammenziehenden, motorisch betriebenen Stellorgans. Der Bandabschnitt wird zum Schutz der Magenwand von einem viskoelastischen Material umgeben, wobei die das viskoelastische Material aufweisende Röhre in einem Ausführungsbeispiel Zwischenwände aufweist, die die Röhre in mehrere mit dem viskoelastischen Material befüllte Kammern unterteilt.

Aufgabe der Erfindung ist es, eine verbesserte Einrichtung der eingangs genannten Art bereitzustellen, bei welcher die Häufigkeit von operativen Eingriffen, welche aufgrund eines Lecks des Bandes notwendig werden, verringert wird. Erfindungsgemäß gelingt dies durch eine Einrichtung mit den Merkmalen des Anspruchs 1.

Bei einem erfindungsgemäßen Band kann im Falle einer Beschädigung der die Durchtrittsöffnung begrenzenden Membran und eines Austritts von Füllmedium aus der ersten Hohlkammer die zweite Hohlkammer zur Befüllung mit dem Füllmedium herangezogen werden. Ein Austausch des Bandes ist daher auch im Falle eines Lecks in der die erste Hohlkammer zur Durchtrittsöffnung hin abdichtenden Membran nicht erforderlich. Insgesamt wird die durchschnittliche Einsatzdauer eines Bandes, bis es die ihm zugedachte Funktion nicht mehr erfüllen kann, wesentlich verlängert.

Günstigerweise umgibt die zweite Hohlkammer im ringförmig geschlossenen Zustand des Bandes die erste Hohlkammer, wobei vorzugsweise die die zweite Hohlkammer auf ihrer der Durchtrittsöffnung zugewandten Seite begrenzende Membran die erste Hohlkammer auf ihrer von der Durchtrittsöffnung abgewandten Seite begrenzt.

Es kann bei einer erfindungsgemäßen Einrichtung vorgesehen sein, dem Patienten zwei Injektionsports oder Pumpeinrichtungen zu implantieren, welche jeweils mit einem Anschlussröhrchen verbunden sind, deren innere Kanäle einerseits mit der ersten, andererseits mit der zweiten Hohlkammer kommunizieren. Wenn die erste Hohlkammer undicht geworden ist, kann mittels des Injektionsportes bzw. der Pumpeinrichtung, der bzw. die an dem mit der zweiten Hohlkammer kommunizierenden Anschlussröhrchen angeschlossen ist, Füllmedium in die zweite Hohlkammer eingebracht werden. Es ist auch denkbar und möglich, dem Patienten nur einen Injektionsport bzw. eine Pumpeinrichtung zu implantieren, wobei daran zunächst das mit der ersten Hohlkammer kommunizierende Anschlussröhrchen angeschlossen ist. Ein Umstecken der Anschlussröhrchen erfordert im Falle seiner Notwendigkeit nur einen vergleichsweise kleinen operativen Eingriff.

Weitere Vorteile und Einzelheiten der Erfindung werden im Folgenden anhand der beiliegenden Zeichnung erläutert. In dieser zeigen:
- Fig. 1: eine Ansicht eines Ausführungsbeispiels einer erfindungsgemäßen Einrichtung in Form eines Magenbandes;
- Fig. 2: eine Seitenansicht, teilweise im Schnitt entlang der Linie A-A von Fig. 1;
- Fig. 3: einen Querschnitt durch das Band entlang der Linie B-B von Fig. 2;
- Fig. 4: einen Schnitt durch einen Abschnitt der Einrichtung entlang der Linie C-C von Fig. 1;
- Fig. 5: einen Schnitt durch einen Abschnitt der Einrichtung entlang der Linie D-D von Fig. 1;
- Fig. 6: eine perspektivische Darstellung dieses Ausführungsbeispiels und
- Fig. 7: eine perspektivische Darstellung eines Injektionsports zum Anschluss an eines der Anschlussröhrchen.

Das in den Figuren dargestellte Ausführungsbeispiel einer erfindungsgemäßen Einrichtung ist als Magenband ausgebildet und weist ein ringförmig um den, in Fig. 2 nur schematisch im Querschnitt angedeuteten, entsprechenden Teil 25 des Gastro-Intestinal-Traktes, hier den Magen, den Mageneingang oder die Speiseröhre zu legendes Band 1 auf. Das Band ist im Bereich seiner beiden Enden mit Teilen einer Verschlusseinrichtung 2 versehen. Die Verschlusseinrichtung weist einen am einen Ende des Bandes 1 angebrachten Fortsatz 3 und einen am anderen Ende des Bandes angebrachten Steg 4 mit einer Einstecköffnung zum Durchstecken des Fortsatzes 3 auf. Zum Schließen der Verschlusseinrichtung 2 wird der Fortsatz 3 durch die Einstecköffnung im Steg 4 durchgezogen, wobei ein Rastvorsprung 5 am Fortsatz 3 mit dem Steg 4 verrastet und die Verschlusseinrichtung gegen ein ungewolltes Öffnen sichert.

Im geschlossenen Zustand der Verschlusseinrichtung 2 umschließt das dann ringförmige Band 1 eine Durchtrittsöffnung 6.

Der Rastvorsprung 5 springt vorzugsweise über die der Durchtrittsöffnung 6 abgewandte Seite und die in Richtung der Achse 7 der Durchtrittsöffnung 6 weisenden Randseiten des Fortsatzes 3 vor, wobei der Rastvorsprung 5 eine vom freien Ende des Fortsatzes 3 aus gesehen konisch sich verbreiternde Form aufweist, um ein Durchziehen durch die Einstecköffnung des Steges 4 zu ermöglichen. Auf der Seite, welche im eingesteckten Zustand den Rand der Einstecköffnung des Steges 4 hintergreift, ist der Rastvorsprung 5 stufenförmig ausgebildet und steht etwa senkrecht vom Fortsatz 3 ab.

Vorzugsweise sind der Fortsatz 3 mit seinem Rastvorsprung 5 und der Steg 4 aus einem elastischen Material, insbesondere Silikon, mit einer solchen Härte und weiters mit einer solchen zusammenwirkenden Geometrie ausgebildet, dass die Verschlusseinrichtung bei einem Aufreißzug, der einen vorgegebenen Grenzwert überschreitet, sich öffnet. Es kann dadurch ein Sicherheitsverschluss bereitgestellt werden, durch welchen ein unzulässiger hoher Druck im umschlossenen Körperorgan vermieden werden kann.

Vom Steg 4 steht eine Zuglasche 8 in eine der Einsteckrichtung des Fortsatzes 3 etwa entgegenstehende Richtung ab, wodurch durch Angriff von medizinischen Instrumenten einerseits an der Zuglasche, andererseits am mit seinem vorderen Ende durch die Einstecköffnung im Steg 4 geführten Fortsatz 3 das Schließen der Verschlusseinrichtung 2 erleichtert wird.

Die an den beiden Enden des Bandes 1 angeordneten Teile der Verschlusseinrichtung 2 können an Pfropfen angebracht sein, welche in die Enden des Bandes ragen, in diese Enden eingeklebt sind und diese Enden verschließen. Auch eine materialeinstückige Ausbildung der Verschlusseinrichtung oder Teilen hiervon mit dem Band 1, insbesondere auf der Seite des Fortsatzes 3 ist denkbar und möglich.

Das Band 1 weist auf seiner von der Durchtrittsöffnung 6 abgewandten Seite einen Rückenabschnitt 9 auf. An dessen beiden Randseiten ist dieser mit in Richtung zur Durchtrittsöffnung 6 abstehenden Fortsätzen 10 versehen (die wesentlich kürzer als die Breite des Rückenabschnitts 9 sind). An diesen Fortsätzen sind zwei elastische Membrane 11, 12 (= Häutchen mit abgrenzender Funktion) angebracht, welche die Fortsätze 10 überbrücken. Die Membrane 11, 12 können beispielsweise mit den Fortsätzen 10 verklebt sein. Auch materialeinstückige Ausbildungen einer oder beider Membrane 11, 12 mit den Fortsätzen 10 und dem Rückenabschnitt 9 sind denkbar und möglich. Hierzu könnten entsprechende Kerne in einer Spritzgussform einerseits zwischen den beiden Membranen 11, 12, andererseits zwischen der Membran 12 und dem Rückenabschnitt 9 angeordnet sein, welche nach dem Spritzen und Entformen des Bandes aus diesem herausgezogen werden. Weiters könnten die Membrane 11 und 12 auch durch zwei ineinander geschobene Schläuche gebildet werden, welche auf der von der Durchtrittsöffnung 6 abgewandten Seite miteinander und mit dem Rückenabschnitt 9 und den Fortsätzen 10 verklebt werden.

Die Fortsätze 10 könnten prinzipiell auch entfallen.

Zwischen der an die Durchtrittsöffnung 6 angrenzenden Membran 11 und der Membran 12 wird eine erste Hohlkammer 13 gebildet, welche sich über zumindest einen Großteil der Länge des Bandes 1 erstreckt. Zwischen der Membran 12 und dem Rückenabschnitt 9 befindet sich eine zweite Hohlkammer 14, welche sich ebenfalls zumindest über einen Großteil der Länge des Bandes 1 erstreckt und radial außerhalb der ersten Hohlkammer 13 angeordnet ist. Das in den Figuren dargestellte Ausführungsbeispiel zeigt die bevorzugte Ausbildung der Hohlkammern 13, 14 über die gesamte Länge des Bandes, wobei die Hohlkammern 13, 14 an den beiden Enden des Bandes durch endseitige Wände 15, 16 verschlossen sind.

Die Membranen 11, 12 bestehen bevorzugterweise ebenso wie der Rückenabschnitt 9 und die gegebenenfalls vorhandenen Fortsätze 10 aus Silikon. Es ist hierbei bevorzugt, dass die elastische Dehnbarkeit der Membranen 11, 12 größer ist als diejenige des Rückenabschnitts 9. Dies kann beispielsweise durch eine Ausbildung des Rückenabschnitts 9 mit einer größeren Dicke als die der Membranen 11, 12 erreicht werden. Auch unterschiedliche Materialhärten des Rückenabschnitts 9 und der Membranen 11, 12 sind denkbar und möglich. Weiters könnte der Rückenabschnitt auch durch eine Verstärkungslage verstärkt sein, die beispielsweise in das Material des Rückenabschnitts eingebettet ist oder auf dessen von der Durchtrittsöffnung 6 abgewandten Seite aufgeklebt ist und sich über die Länge des Bandes erstreckt. Diese Verstärkungslage kann hierbei in Längsrichtung und vorteilhafterweise auch in Querrichtung über ihre gesamte Ausdehnung durchgehende Fäden aufweisen und kann insbesondere als Rechteckgewebe, beispielsweise aus einem Kunststoffmaterial ausgebildet sein.

Durch die wenig elastische oder unelastische Ausbildung des Rückenabschnitts wird dessen Ausdehnung bei der Befüllung des Bandes mit einem Füllmaterial verhindert.

Die Einrichtung weist weiters zwei Anschlussröhrchen 17, 18 auf, deren innere Kanäle 19, 20 einerseits mit der ersten Hohlkammer 13, andererseits mit der zweiten Hohlkammer 14 kommunizieren. Hierfür sind Anschlussstutzen 21, 22 am Band 1 angebracht, beispielsweise einstückig mit dem Band 1 ausgebildet, welche einerseits in die erste Hohlkammer 13, andererseits in die zweite Hohlkammer 14 münden und in welche die Anschlussröhrchen 17, 18 mit Endabschnitten eingeklebt sind. Die Anschlussstutzen 21, 22 gehen in einem mittleren Bereich des Bandes 1 von diesem aus.

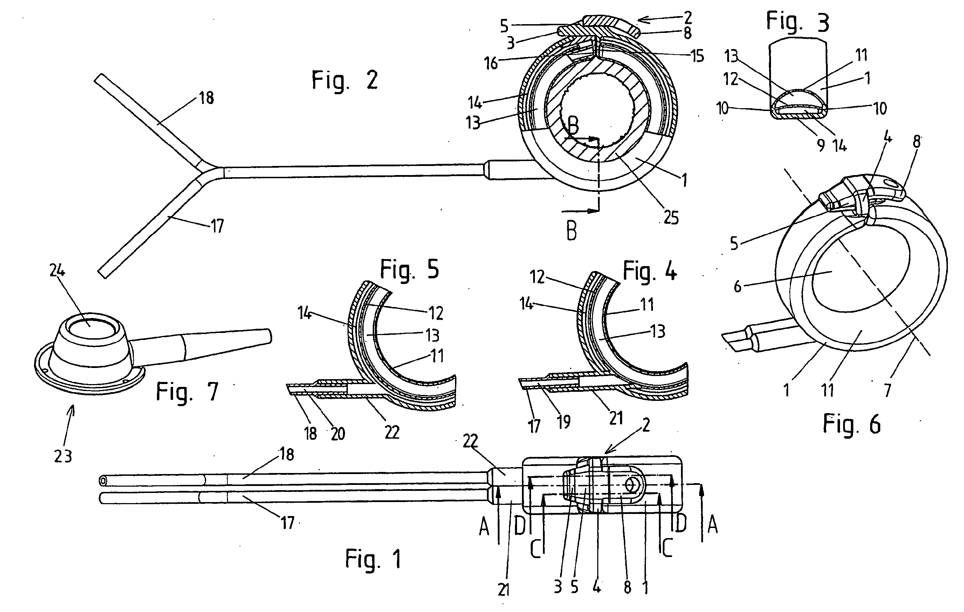

An die anderen Enden der Anschlussröhrchen kann beispielsweise jeweils ein Injektionsport 23 angeschlossen sein, wie dieser in Fig. 7 dargestellt ist. Der Injektionsport 23 weist in herkömmlicher Weise eine Membran 24 auf, durch welche die Nadel einer mit dem Füllmaterial, insbesondere steriles Wasser, befüllte Spritze durch die Haut des Patienten hindurch durchgestochen werden kann, um die entsprechende Hohlkammer 13, 14 des Bandes 1 mit der gewünschten Menge von Füllmaterial zu befüllen und dadurch den Öffnungsquerschnitt der Durchtrittsöffnung 6 in der gewünschten Weise einzustellen. Die relativ dick ausgebildete Membran 24 schließt sich nach dem Herausziehen der Nadel der Spritze wiederum dicht.

Nach der Platzierung des mittels der Verschlusseinrichtung 2 ringförmig geschlossenen Bandes um das Körperorgan wird zunächst die erste Hohlkammer 13 mit der gewünschten Menge von Füllmaterial gefüllt, so dass sich die Membran 11 entsprechend in Richtung zur Achse 7 ausdehnt, bis die Größe der Durchtrittsöffnung 6 und somit die Größe der Durchlassöffnung des Teils 25 des Gastro-Intestinal-Traktes auf den gewünschten Wert eingestellt ist. Die Membran 12 legt sich hierbei an den Rückenabschnitt 9 des Bandes 1 an.

Falls die Hohlkammer 13 aufgrund eines Lecks in der Membran 11 undicht wird, kann das Füllmaterial aus der Hohlkammer 13 austreten, so dass sich die Membran 11 entspannt und die Durchtrittsöffnung 6 vergrößert und die Funktion des Bandes 1 verloren geht. In diesem Fall kann die zweite Hohlkammer 14 mit Füllmaterial befüllt werden, so dass sich nunmehr die Membran 12 in Richtung zur Achse 7 entsprechend ausdehnt, wobei sie die Membran 11 mitnimmt. Über die Befüllung der Hohlkammer 14 und der Ausdehnung der Membran 12 kann die Größe der Durchtrittsöffnung somit auch noch im Falle eines Lecks in der Membran 11 wunschgemäß eingestellt werden. Die durchschnittliche Gebrauchsdauer der Einrichtung wird dadurch insgesamt wesentlich verlängert, so dass den Patienten operative Eingriffe erspart werden.

Anstelle eines dargestellten Injektionsports könnte auch eine Pumpeinrichtung zur Befüllung der jeweiligen Hohlkammer 13, 14 an das Anschlussröhrchen 17 bzw. 18 angeschlossen sein. Eine solche könnte ein Reservoir mit Füllmaterial aufweisen, dessen Volumen veränderbar ist. Wenn die Einrichtung als öffen- und schließbarer Verschluss ausgebildet ist, beispielsweise als Analband, so müsste das Reservoir in bekannter Weise zwischen zwei Größen seines Volumens umschaltbar sein, welche dem geschlossenen und dem geöffneten Zustand der Einrichtung entsprechen.

Unterschiedliche Modifikationen der beschriebenen Ausführungsbeispiele sind denkbar und möglich. Beispielsweise könnten die Membrane 11, 12 auch mehrschichtig ausgebildet sein. Als Füllmedium könnten auch andere Fluide, insbesondere Flüssigkeiten, vorgesehen sein, welche beispielsweise eine höhere Viskosität als Wasser aufweisen könnten.

### Legende

### zu den Hinweisziffern:

- 1: Band
- 2: Verschlusseinrichtung
- 3: Fortsatz
- 4: Steg
- 5: Rastvorsprung
- 6: Durchtrittsöffnung
- 7: Achse
- 8: Zuglasche
- 9: Rückenabschnitt
- 10: Fortsatz
- 11: Membran
- 12: Membran
- 13: Hohlkammer
- 14: Hohlkammer
- 15: Wand
- 16: Wand
- 17: Anschlussröhrchen
- 18: Anschlussröhrchen
- 19: Kanal
- 20: Kanal
- 21: Anschlussstutzen
- 22: Anschlussstutzen
- 23: Injektionsport
- 24: Membran
- 25: Teil des Gastro-Intestinal-Traktes

## Patentansprüche

1. Einrichtung zur Erzeugung einer künstlichen Verengung im Gastro-Intestinal-Trakt mit einem ringförmig um den jeweiligen Teil des Gastro-Intestinal-Traktes legbaren Band (1) und einer Verschlusseinrichtung (2) zum Verbinden der Endbereiche des ringförmig um den Teil des Gastro-Intestinal-Traktes gelegten Bandes (1), welches im geschlossenen Zustand der Verschlusseinrichtung (2) eine Durchtrittsöffnung (6) umschließt, wobei das Band (1) eine mit einem Füllmedium befüllbare erste Hohlkammer (13) aufweist, die sich über zumindest einen Großteil der Länge des Bandes (1) erstreckt und auf ihrer der Durchtrittsöffnung (6) zugewandten Seite von einer Membran (11) begrenzt ist, mittels der durch Befüllung der Hohlkammer (13) die Durchtrittsöffnung (6) verkleinerbar ist, **dadurch gekennzeichnet, dass** das Band weiters eine von dieser ersten Hohlkammer (13) getrennte zweite mit einem Füllmedium befüllbare Hohlkammer (14) aufweist, die sich über zumindest einen Großteil der Länge des Bandes (1) erstreckt und die auf ihrer der Durchtrittsöffnung (6) zugewandten Seite von einer weiteren Membran (12) begrenzt ist, mittels welcher durch Befüllung der zweiten Hohlkammer (14) die Durchtrittsöffnung (6) verkleinerbar ist.

2. Einrichtung nach Anspruch 1, **dadurch gekennzeichnet, dass** die zweite Hohlkammer (14) im ringförmig geschlossenen Zustand des Bandes (1) die erste Hohlkammer (13) umgibt.

3. Einrichtung nach Anspruch 1 oder Anspruch 2, **dadurch gekennzeichnet, dass** die die zweite Hohlkammer (14) auf ihrer der Durchtrittsöffnung (6) zugewandten Seite begrenzende Membran (12) die erste Hohlkammer (13) auf ihrer von der Durchtrittsöffnung (6) abgewandten Seite begrenzt.

4. Einrichtung nach einem der Ansprüche 1 bis 3, **dadurch gekennzeichnet, dass** die beiden Membranen (11, 12) elastisch dehnbar sind, vorzugsweise aus Silikon bestehen.

5. Einrichtung nach einem der Ansprüche 1 bis 4, **dadurch gekennzeichnet, dass** das Band (1) auf ihrer von der Durchtrittsöffnung (6) abgewandten Seite einen im Vergleich zu den Membranen (11, 12) weniger dehnbaren Rückenabschnitt (9) aufweist, welcher vorzugsweise zumindest teilweise von einem Silikon gebildet wird.

6. Einrichtung nach Anspruch 5, **dadurch gekennzeichnet, dass** die Membranen (11, 12) am Rückenabschnitt (9) oder an von diesem an seinen beiden Randseiten in Richtung zur Durchtrittsöffnung (6) abstehenden Fortsätzen (10) angebracht sind.

7. Einrichtung nach einem der Ansprüche 1 bis 6, **dadurch gekennzeichnet, dass** das Band an seinen beiden Enden die Hohlkammern (13, 14) verschlie-ßende Wände (15, 16) aufweist.

8. Einrichtung nach einem der Ansprüche 1 bis 7, **dadurch gekennzeichnet, dass** die Einrichtung erste und zweite Anschlussröhrchen (17, 18) mit inneren Kanälen (19, 20) aufweist, wobei der innere Kanal (19) des einen Anschlussröhrchens (17) mit der ersten Hohlkammer (13) und der innere Kanal (20) des anderen Anschlussröhrchens (18) mit der zweiten Hohlkammer (14) kommuniziert.

9. Einrichtung nach Anspruch 8, **dadurch gekennzeichnet, dass** die Anschlussröhrchen (17, 18) an Anschlussstutzen (21, 22) angebracht sind, von denen der eine Anschlussstutzen (21) in die erste Hohlkammer (13) und der andere Anschlussstutzen (22) in die zweite Hohlkammer (14) mündet.

## Claims

1. A device for the production of an artificial constriction in the gastrointestinal tract, having a band (1) placeable in an annular manner around the specific part of the gastrointestinal tract and a closing device (2) to connect the end regions of the band (1) placed in an annular manner around the part of the gastrointestinal tract, which band (1) in the closed state of the closing device (2) surrounds an opening (6), wherein the band (1) has a first hollow chamber (13) fillable with a filling medium, which extends over at least a major part of the length of the band (1) and which on its side facing the opening (6) is bounded by a diaphragm (11) by means of which the opening (6) can be reduced in size through filling of the hollow chamber (13), **characterised in that** the band furthermore has a second hollow chamber (14) fillable with a filling medium, which is separate from this first hollow chamber (13) and extends over at least a major part of the length of the band (1) and which on its side facing the opening (6) is bounded by a further diaphragm (12) by means of which the opening (6) can be reduced in size through filling of the second hollow chamber (14).

2. A device according to claim 1, **characterised in that** the second hollow chamber (14) surrounds the first hollow chamber (13) in the annularly closed state of the band (1).

3. A device according to claim 1 or claim 2, **characterised in that** the diaphragm (12) bounding the second hollow chamber (14) on its side facing the opening (6) bounds the first hollow chamber (13) on its side remote from the opening (6).

4. A device according to any one of claims 1 to 3, **characterised in that** the two diaphragms (11, 12) are resiliently extensible, preferably are composed of silicone.

5. A device according to any one of claims 1 to 4, **characterised in that** on its side remote from the opening (6), the band (1) has a back portion (9) of less extensibility than the diaphragms (11, 12), which back portion (9) is preferably at least partially formed by a silicone.

6. A device according to claim 5, **characterised in that** the diaphragms (11, 12) are attached to the back portion (9) or to prolongations (10) projecting from the two edge sides of the latter in the direction of the opening (6).

7. A device according to any one of claims 1 to 6, **characterised in that** both ends of the band have walls (15, 16) closing the hollow chambers (13, 14).

8. A device according to any one of claims 1 to 7, **characterised in that** the device has first and second connection tubes (17, 18) with inner channels (19, 20), wherein the inner channel (19) of one connection tube (17) communicates with the first hollow chamber (13) and the inner channel (20) of the other connection tube (18) communicates with the second hollow chamber (14).

9. A device according to claim 8, **characterised in that** the connection tubes (17, 18) are attached to connecting pieces (21, 22) of which one connecting piece (21) opens into the first hollow chamber (13) and the other connecting piece (22) opens into the second hollow chamber (14).

## Revendications

1. Dispositif pour produire une constriction artificielle du tract gastro-intestinal avec un bandeau (1) annulaire pouvant être posé sur la partie souhaitée du tract gastro-intestinal et un obturateur (2) pour relier la zone d'extrémité du bandeau (1) annulaire posé autour de la partie du tract gastro-intestinal qui, en état fermé de l'obturateur (2), entoure en le fermant un orifice de passage (6), le bandeau (1) présentant une première chambre creuse (13) pouvant être remplie par un moyen de remplissage, chambre qui s'étend sur au moins une grande partie de la longueur du bandeau (1) et qui est limitée de son côté associé à l'ouverture de passage (6) par une membrane (11) à l'aide de laquelle par le remplissage de la chambre creuse (13) on rétrécit l'ouverture de passage (6),
**caractérisé en ce que**
le bandeau présente plus loin, une seconde chambre creuse (14) pouvant être remplie avec un moyen de remplissage séparé de la première chambre creuse (13), cette chambre (14) s'étendant sur au moins une grande partie de la longueur du bandeau (1) et étant limitée de son côté associé à l'ouverture de passage par une membrane (12) à l'aide de laquelle par le remplissage de la seconde chambre creuse (14) on rétrécit l'ouverture de passage (6).

2. Dispositif selon la revendication 1,
**caractérisé en ce que**
la seconde chambre creuse (14) entoure, en état fermé du bandeau annulaire (1), la première chambre creuse (13).

3. Dispositif selon la revendication 1 ou 2,
**caractérisé en ce que**
sur sa membrane (12) limitant le côté associé à l'ouverture de passage (6), la seconde chambre creuse (14) limite la première chambre creuse (13) sur son côté opposé à l'ouverture de passage (6),

4. Dispositif selon les revendications 1 à 3,
**caractérisé en ce que**
les deux membranes (11, 12) sont extensibles élastiquement et se composent de préférence de silicone.

5. Dispositif selon les revendications 1 ou 4,
**caractérisé en ce que**
le bandeau (1) présente un segment arrière (9) du côté opposé à l'ouverture de passage (6) moins extensible que les membranes (11, 12), qui est de préférence au moins partiellement en silicone.

6. Dispositif selon la revendication 5,
**caractérisé en ce que**
les membranes (11, 12) sont montées sur le segment arrière ou à des parties en saillie 10 sur ces deux côtés périphériques s'étendant en direction de l'ouverture de passage 6.

7. Dispositif selon les revendications 1 à 6,
**caractérisé en ce que**
le bandeau présente des parois de fermeture (15, 16) à ses deux extrémités de chambres creuses (13, 14).

8. Dispositif selon les revendications 1 à 7,
**caractérisé par**
un premier et un second tuyau (17, 18) de branchement avec des canaux intérieurs (19, 20), le canal intérieur (19) de l'un des tuyaux (17) communiquant avec la première chambre creuse (13) et le canal intérieur (20) de l'autre tuyau (18) communiquant avec la seconde chambre creuse (14).

9. Dispositif selon la revendication 8,
**caractérisé en ce que**
les tuyaux de raccordement (17, 18) sont branchés à des embouts de branchement (20, 21, 22), un embout (21) aboutissant dans la première chambre creuse (13) et l'autre embout (22) aboutissant dans la seconde chambre creuse (14).
